# EUROPEAN PATENT SPECIFICATION

(11) **EP 3 946 289 B1**
(45) Date of publication and mention of the grant of the patent: **09.07.2025**
(21) Application number: 20726900.2
(22) Date of filing: 02.04.2020
(51) Int. Cl.: A61K 31/015, A61K 31/045, A61K 31/07, A61K 31/203, A61K 31/22, A61K 31/23, A61K 35/28, A61P 43/00

(54) **VITAMIN A FOR USE IN THE TREATMENT OF TENDON INJURY OR LIGAMENT INJURY**
VITAMIN A ZUR VERWENDUNG BEI DER BEHANDLUNG VON SEHNENVERLETZUNG ODER BANDVERLETZUNG
VITAMINE A POUR UTILISATION DANS LE TRAITEMENT D'UNE LÉSION D'UN TENDON OU D'UNE LÉSION D'UN LIGAMENT

(30) Priority: 02.04.2019 GB 201904639; 10.04.2019 GB 201905089
(43) Date of publication of application: 09.02.2022
(73) Proprietor: Regenall Limited, London W1G 9QJ (GB)
(72) Inventor: NOORDEEN, Mohamed Hamza, London W1G 9QJ (GB)
(74) Representative: Reddie & Grose LLP
(86) International application number: PCT/GB2020/050886
(87) International publication number: WO 2020/201763

(56) References cited:
- WO-A1-2018/034314
- CN-A- 107 753 935
- CN-A- 107 929 098
- CN-A- 108 853 146
- ES-A1- 2 387 109
- JP-A- 2004 059 438
- US-B1- 6 506 801
- GREENWALD D P ET AL: "Zone II flexor tendon repair: Effects of vitamins A, E, @b-carotene", JOURNAL OF SURGICAL RESEARCH, ACADEMIC PRESS INC., SAN DIEGO, CA, US, vol. 49, no. 1, 1 July 1990 (1990-07-01), pages 98 - 102, XP023022166, ISSN: 0022-4804, [retrieved on 19900701], DOI: 10.1016/0022-4804(90)90117-K
- ANONYMOUS: "Puritan's Pride BETA-CAROTENE 25.000 IU 100GELS | Vitaminen | Body&Fit NL", 16 August 2018 (2018-08-16), pages 1 - 6, XP055895673, Retrieved from the Internet <URL:https://www.bodyandfit.com/nl-nl/Producten/Vitamines-en-supplementen-/Vitaminen/Beta-Carotene-25-000-IU/p/17661?review-page=2>
- BERYL SHUTTLEWORTH: "Vitamin A for Horses - How much is too much?", 1 July 2016 (2016-07-01), pages 1 - 3, XP093064465, Retrieved from the Internet <URL:https://www.horseandpethealth.com/vitamin-a-for-horses/> [retrieved on 20230717]

## Description

### TECHNICAL FIELD

This invention relates to compounds and multiple-dose formulations for use in the treatment of tissue damage or injury, in particular, for example, tendon injury. Methods of treatment of tissue damage or injury using the compounds or multiple-dose formulations are also described.

### BACKGROUND

Tissue repair encompasses two separate processes: regeneration and replacement. Regeneration refers to a type of healing in which new growth completely restores portions of damaged tissue to their normal state. Replacement refers to a type of healing in which severely damaged or non-regenerable tissues are repaired by the laying down of connective tissue (or glial tissue in the central nervous system, for example the brain or spinal cord), a process commonly referred to as scarring. Whilst some types of tissue injury (such as minor paper cuts) can sometimes be healed in such a way that no permanent damage remains, most tissue repair consists of both regeneration and replacement. Tissue repair may restore some of the original structures of the damaged tissue (such as epithelial layers), but may also result in structural abnormalities that impair function (such as the scar formed in the healing of a myocardial infarction).

The extent to which regeneration and/or replacement occurs following injury depends, in part, on the type of tissue involved. Certain tissues of the body are more capable of cellular proliferation (and hence regeneration) than others. There are three types of tissues: continuously dividing tissues, quiescent tissues, and non-dividing tissues. Continuously dividing tissues are comprised of cells that are constantly proliferating in order to replace dead or sloughed-off cells. Examples of such tissues include epithelia (such as skin, gastrointestinal epithelium and salivary gland tissue) and hematopoietic tissues. These tissues contain pools of stem cells, which have enormous proliferative and self-renewing ability, and which give rise to more than one type of cell. Replicating asymmetrically, each stem cell gives rise to one daughter cell that differentiates and matures and another daughter cell that remains undifferentiated and capable of beginning another self-renewing cycle. Quiescent tissues are composed of cells that normally exist in a non-dividing state but may enter the cell cycle in response to certain stimuli, such as cell injury. Tissues falling into this category include parenchymal cells of the liver, kidney and pancreas, mesenchymal cells such as fibroblasts and smooth muscle cells, endothelial cells and lymphocytes. A few types of tissue are composed of cells that have left the cell cycle permanently, and are therefore unable to proliferate. These non-dividing tissues include cardiac and skeletal muscle. Tissue repair in these tissues always leaves permanent evidence of injury, such as a scar.

In adults, most cells, such as myocytes, adipocytes, skin cells and neurons, are in the non-dividing state, i.e. terminally-differentiated. Terminal differentiation is the process by which cells during the course of development become specialized, taking on specific structural, functional, and biochemical properties and roles. The brain is composed of both non-dividing and dividing cells. Specifically, differentiated neurons are in a post-mitotic state and cannot re-enter the cell cycle. Glial cells (e.g., astrocytes, oligodendrocytes, and microglia) are in either a proliferative or non-proliferative state, depending on their differentiation status and possible re-entry into the cell cycle.

Spinal cord injury (SCI) is a major cause of long-term physical impairment. It results in disruption of cord microstructure and is followed by limited neuronal regeneration and insufficient functional recovery in adult mammals. In some cases, nerve roots may be torn or avulsed from the spinal cord, leading to degeneration of nerve fibres, death of nerve cells and a breakdown of connections within the spinal cord. Root avulsion has been associated with an overall poor clinical outcome. Scar tissue formation in spinal cord injury is reviewed in O'Shea et al. Cell biology of spinal cord injury and repair, J. Clin. Invest. 2017, 127(9), 3259-3270. Briefly, after severe SCI, and in response to changes in the local microenvironment, astrocytes, the most abundant glial cells in the central nervous system (CNS) transform into reactive astrocytes and undergo dramatic morphological changes as well as massive variations in gene expression. Reactive astrocytes, the major component of glial scars, along with other cells in the spinal cord and blood-borne cells leaking from the damaged blood-spinal cord barrier, participate in the process of scar formation. Mature spinal cord injury lesions comprise: (a) a central non-neural lesion core, often referred to as a fibrotic scar, (b) an astroglial scar border that intimately surrounds the lesion core; and (c) a surrounding zone of viable neural tissue that is functional but is demarcated by the presence of reactive glia. Spinal cord injury can be caused by single large lesions or multiple small lesions that span the entire spinal cord.

Current attempts to manage spinal cord injuries involve maintenance of spinal cord nerve cells and regeneration of nerve fibres within the spinal cord, i.e. central nervous regeneration. Medullary implantation of peripheral nerve grafts or re-implantation of avulsed ventral or motor roots into the spinal cord, if performed early after the injury, can curtail some neuronal loss and promote some regrowth of new motor or autonomic axons through spinal cord tissue (central nerve fibre regeneration) and further into the peripheral nerves. However, it has not been possible to reconstruct avulsed dorsal or sensory roots as these nerve fibres are prevented or inhibited from entering into the spinal cord. Today, most patients are not treated at all or receive non-curative palliative procedures which do not give acceptable functional return or pain alleviation. There are currently no effective treatments for spinal cord injuries, which can result in loss of feeling and control of the body below the level of the spinal cord injury.

Stem cells have been extensively studied as neuroregenerative and neuroprotective agents for the treatment of SCI (reviewed by Gazdic et al., Stem Cells Therapy for Spinal Cord Injury, Int. J. Mol. Sci. 2018, 19, 1039). Treatments for mobilisation of endogenous stem cell population are considered to provide a promising therapeutic approach. Results of preclinical studies indicate that application of stem cell-derived progenitors significantly reduces neurological disability in most severe SCIs. However, stem cell transplantation alone is not sufficient to bridge a spinal cord lesion.

As with SCI, there are currently no effective treatments for other neurological injuries, such as brain injury or peripheral nerve injury.

Tendon injuries are some of the most common orthopaedic problems, and cause substantial pain, disability, and time off work. Whilst many tendon injuries are acute, a large number are chronic, degenerative conditions. In either case, repair results in the formation of a fibrovascular scar that never attains the characteristics of normal tendon. Tendon healing is characterised by the formation of fibrovascular scar tissue, as tendon has very little intrinsic regenerative capacity. The molecular mechanisms resulting in scar tissue formation after tendon injuries are not well understood (as reviewed in Schneider et al. Rescue plan for Achilles: Therapeutics steering the fate and functions of stem cells in tendon wound healing; Advanced Drug Delivery Reviews 129 2018 352-375*).* Briefly, in the first few days after injury a blood clot forms that serves as a preliminary scaffold for invading cells followed by a more robust vascular network which is essential for the survival of tenocytes engaged in the synthesis of new fibrous tissue. Thereafter, fibroblasts are recruited to the injured site and produce initially disorganised extracellular matrix components. Following this a remodelling stage commences characterised by tissue changes resulting in a more fibrous appearance and eventually a scar-like tendon tissue can be observed.

Current biologic treatment strategies have not achieved tendon regeneration but include the use of extracellular matrix patches to provide a scaffold for new cell growth and differentiation (as reviewed in Galatz et al. Tendon Regeneration and Scar Formation: The Concept of Scarless Healing, J. Orthop. Res. 2015, 33(6) 823-831)*.* Platelet rich plasma which comprises a multitude of growth factors normally involved in repair processes has also been investigated for use in tendon repair. However, there is no evidence that either strategy induces tendon regeneration. Tendon injuries are also a particular problem in horses. Spanish patent application 2387109 A1 describes a pharmaceutical spray comprising a therapeutically effective amount of *Calendula officinalis,* a therapeutically effective amount of vitamin A and a therapeutically effective amount of vitamin E for the topical treatment of pain.

There is, therefore, an urgent need to provide more effective treatments for tissue injury, including neurological injury such as spinal cord injury, brain injury, and peripheral nerve injury, and tendon injury, in particular treatments which provide improvements in functional recovery following injury.

### SUMMARY

The applicant has recognised that scar tissue formation, for example due to accumulation of extracellular matrix proteins such as collagen and/or glycosaminoglycans, prevents improvement in functionality of an injured tissue even after treatment with stem cells or surgery. In fact, administration of stem cells to an injured tissue, for example in SCI, may even increase the formation of scar tissue at the injury site, thereby impairing the repair process and reducing functional recovery. The applicant has appreciated that inhibition of scar tissue formation following tissue injury is a key aspect of the repair process, and in particular is a necessary prerequisite for successful tissue regeneration. The applicant has also recognised that vitamin A may be used to inhibit scar tissue formation, and may thus be used in the effective treatment of tissue injury, including neurological injury (such as spinal cord injury, brain injury, or peripheral nerve injury) and soft tissue injury (such as tendon injury).

### DESCRIPTION

The present invention is defined by the claims. Any subject-matter falling outside the scope of the claims is provided for information only. Methods of treatment of the human body by therapy, referred to in this description, are not part of the present invention as such, but are described here in relation to compounds or pharmaceutical compositions for use in said methods of treatment of the human body by therapy, according to the present invention.

There is provided according to the invention vitamin A for use in the treatment of tissue damage in a subject following an injury to the subject, wherein the injury comprises a soft tissue injury, wherein the soft tissue injury is a tendon injury or a ligament injury, wherein the vitamin A is to be administered systemically by oral or intravenous administration to the subject, and wherein:
a) the subject is a human subject and the vitamin A is for administration to the subject at a dose of >10,000 to 100,000 IU vitamin A per day; or
b) the subject is a horse and the vitamin A is for administration to the horse at a dose of 250 to 500 IU/kg body weight per day.

According to the disclosure there is provided vitamin A for use in inhibition of scar tissue formation in a subject.

There is also provided according to the disclosure use of vitamin A in the manufacture of a medicament for the inhibition of scar tissue formation in a subject.

There is further provided according to the disclosure a method of inhibiting scar tissue formation in a subject comprising administering to the subject an effective amount of vitamin A.

Particular embodiments of the disclosure relate to inhibition of scar tissue formation occurring other than as a result of infection, for example as a result of injury.

There is also provided according to the disclosure vitamin A for use in inhibition of scar tissue formation in a subject, following an injury to the subject, for the treatment of the injury.

There is also provided according to the disclosure use of vitamin A in the manufacture of a medicament for the inhibition of scar tissue formation in a subject, following an injury to the subject, for the treatment of the injury.

There is further provided according to the disclosure a method of inhibiting scar tissue formation in a subject, following an injury to the subject, for the treatment of the injury comprising administering to the subject an effective amount of vitamin A.

There is also provided according to the disclosure vitamin A for use in the treatment of an injury to a subject.

There is also provided according to the disclosure use of vitamin A in the manufacture of a medicament for the treatment of an injury to a subject.

There is further provided according to the disclosure a method of treating an injury to a subject, comprising administering to the subject an effective amount of vitamin A.

There is also provided according to the disclosure use of vitamin A in the manufacture of a medicament for the treatment of tissue damage in a subject.

There is further provided according to the disclosure a method of treating tissue damage in a subject comprising administering to the subject an effective amount of vitamin A.

The tissue damage is tissue damage resulting from an injury to the subject.

Vitamin A is the name of a group of fat-soluble retinoids, including retinol, retinal, and retinyl esters. There are two different categories of vitamin A. The first category, preformed vitamin A, comprises retinol and its esterified form, retinyl ester. The second category, provitamin A, comprises provitamin A carotenoids such as alpha-carotene, beta-carotene and beta-cryptoxanthin. Both retinyl esters and provitamin A carotenoids are converted to retinol, which is oxidized to retinal and then to retinoic acid. Both provitamin A and preformed vitamin A are known be metabolized intracellularly to retinal and retinoic acid, the bioactive forms of vitamin A.

Vitamin A for use according to the invention may be an isolated form of vitamin A. An isolated form of vitamin A is any form of vitamin A found in the diet or a metabolized form thereof. For example, vitamin A may be isolated from fish liver oil. Vitamin A may comprise a preformed vitamin A such as retinol or a retinyl ester. Retinyl esters include retinyl acetate and retinyl palmitate. Vitamin A may comprise a provitamin A, such as a provitamin A carotenoid including alpha-carotene, beta-carotene or beta-cryptoxanthin. Vitamin A may comprise a bioactive form of vitamin A such as retinal or retinoic acid.

Vitamin A is available for human consumption in multivitamins and as a stand-alone supplement, often in the form of retinyl acetate or retinyl palmitate. A portion of the vitamin A in some supplements is in the form of beta-carotene and the remainder is preformed vitamin A; others contain only preformed vitamin A or only beta-carotene. Supplement labels usually indicate the percentage of each form of the vitamin. The amounts of vitamin A in stand-alone supplements range widely. Multivitamin supplements typically contain 2,500 to 10,000 international units (IU) vitamin A, often in the form of both retinol and beta-carotene.

Vitamin A is listed on food and supplement labels in international units (IUs). However, Recommended Dietary Allowance (RDA) (average daily level of intake sufficient to meet the nutrient requirements of nearly all (97%-98%) healthy individuals) for vitamin A is given as micrograms (µg; mcg) of retinol activity equivalents (RAE) to account for the different bioactivities of retinol and provitamin A carotenoids (see Table 1). Because the body converts all dietary sources of vitamin A into retinol, 1 mcg of physiologically available retinol is equivalent to the following amounts from dietary sources: 1 mcg of retinol, 12 mcg of beta-carotene, and 24 mcg of alpha-carotene or beta-cryptoxanthin. From dietary supplements, the body converts 2 mcg of beta-carotene to 1 mcg of retinol.

Conversion rates between mcg RAE and IU are as follows:
- 1 IU retinol = 0.3 mcg RAE;
- 1 IU beta-carotene from dietary supplements = 0.15 mcg RAE;
- 1 IU beta-carotene from food = 0.05 mcg RAE; and
- 1 IU alpha-carotene or beta-cryptoxanthin = 0.025 mcg RAE.

An RAE cannot be directly converted into an IU without knowing the source(s) of vitamin A. For example, the RDA of 900 mcg RAE for adolescent and adult men is equivalent to 3,000 IU if the food or supplement source is preformed vitamin A (retinol). However, this RDA is also equivalent to 6,000 IU of beta-carotene from supplements, 18,000 IU of beta-carotene from food, or 36,000 IU of alpha-carotene or beta-cryptoxanthin from food. So a mixed diet containing 900 mcg RAE provides between 3,000 and 36,000 IU of vitamin A, depending on the foods consumed.

| Table 1: Recommended Dietary Allowances (RDAs) for Vitamin A | | | | |
|---|---|---|---|---|
| **Age** | **Male** | **Female** | **Pregnancy** | **Lactation** |
| 0-6 months* | 400 mcg RAE | 1400 mcg RAE | | |
| 7-12 months* | 500 mcg RAE | 1500 mcg RAE | | |
| 1-3 years | 300 mcg RAE | 300 mcg RAE | | |
| 4-8 years | 400 mcg RAE | 1400 mcg RAE | | |
| 9-13 years | 600 mcg RAE | 600 mcg RAE | | |
| 14-18 years | 900 mcg RAE | 700 mcg RAE | 750 mcg RAE | 1,200 mcg RAE |
| 19-50 years | 900 mcg RAE | 700 mcg RAE | 770 mcg RAE | 1,300 mcg RAE |
| 51+ years | 900 mcg RAE | 700 mcg RAE | | |

| | | | | |
|---|---|---|---|---|
| * Adequate Intake (AI), equivalent to the mean intake of vitamin A in healthy, breastfed infants. Source: National Institutes of Health, Vitamin A, Fact Sheet for Health Professionals, as updated 5 October 2018 | | | | |

The Food and Nutrition Board (FNB) at the Institute of Medicine of the National Academies (formerly National Academy of Sciences) has established tolerable Upper Intake Level (UL) (maximum daily intake unlikely to cause adverse health effects) for preformed vitamin A that apply to both food and supplement intakes. The FNB based these ULs on the amounts associated with an increased risk of liver abnormalities in men and women, teratogenic effects, and a range of toxic effects in infants and children. The FNB has not established ULs for beta-carotene and other provitamin A carotenoids.

| Table 2: Tolerable Upper Intake Levels (ULs) for Preformed Vitamin A* | | | | |
|---|---|---|---|---|
| **Age** | **Male** | **Female** | **Pregnancy** | **Lactation** |
| 0-12 months | 600 mcg RAE (2,000 IU) | 600 mcg RAE (2,000 IU) | | |
| 1-3 years | 600 mcg RAE (2,000 IU) | 600 mcg RAE (2,000 IU) | | |
| 4-8 years | 900 mcg RAE (3,000 IU) | 900 mcg RAE (3,000 IU) | | |
| 9-13 years | 1,700 mcg RAE (5,667 IU) | 1,700 mcg RAE (5,667 IU) | | |
| 14-18 years | 2,800 mcg RAE (9,333 IU) | 2,800 mcg RAE (9,333 IU) | 2,800 mcg RAE (9,333 IU) | 2,800 mcg RAE (9,333 IU) |
| 19+ years | 3,000 mcg RAE (10,000 IU) | 3,000 mcg RAE (10,000 IU) | 3,000 mcg RAE (10,000 IU) | 3,000 mcg RAE (10,000 IU) |

| | | | | |
|---|---|---|---|---|
| Source: National Institutes of Health, Vitamin A, Fact Sheet for Health Professionals, as updated 5 October 2018 * These ULs, expressed in mcg and in IUs (where 1 mcg = 3.33 IU), only apply to products from animal sources and supplements whose vitamin A comes entirely from retinol or ester forms, such as retinyl palmitate. However, many dietary supplements (such as multivitamins) do not provide all of their vitamin A as retinol or its ester forms. For example, the vitamin A in some supplements consists partly or entirely of beta-carotene or other provitamin A carotenoids. In such cases, the percentage of retinol or retinyl ester in the supplement should be used to determine whether an individual's vitamin A intake exceeds the UL. For example, a supplement labeled as containing 10,000 IU of vitamin A with 60% from beta-carotene (and therefore 40% from retinol or retinyl ester) provides 4,000 IU of preformed vitamin A. That amount is above the UL for children from birth to 13 years but below the UL for adolescents and adults. | | | | |

It will be appreciated that use of a natural vitamin for tissue repair is particularly advantageous because of its known safety profile.

Preferably vitamin A for use in inhibition of scar tissue formation in a subject according to the disclosure comprises a high dose of vitamin A.

A high dose of vitamin A is considered to be a dose that exceeds a UL for the subject. Examples of high doses of vitamin A include: >10,000 IU to 100,000 IU vitamin A per day; about 25,000 to 100,000 IU vitamin A per day; about 50,000 to 100,000 IU vitamin A per day; or about 75,000 to 100,000 IU vitamin A per day, in particular of preformed vitamin A.

Vitamin A may be administered to the subject once per day, twice per day, three times per day, four times per day, or five times per day.

Vitamin A may be administered to the subject for at least 3 days for example for at least a week, for at least a month, or for at least 6 months from the day of first administration to the subject.

Prolonged exposure to high doses of vitamin A may lead to hypervitaminosis A. Thus, it may be preferred to limit administration of high doses of vitamin A to the subject for up to 6 years, or up to 6 months, from the day of first administration to the subject.

Optionally for an adult human subject (>18 years old), the subject may be administered up to 100,000 IU vitamin A (in particular of preformed vitamin A) per day for up to 6 months. For example, >10,000 IU to 100,000 IU vitamin A per day; about 25,000 to 100,000 IU vitamin A per day; about 50,000 to 100,000 IU vitamin A per day; or about 75,000 to 100,000 IU vitamin A per day (in particular of preformed vitamin A) for up to 6 months.

Optionally for an adult human subject (>18 years old), the subject may be administered up to 25,000 IU vitamin A per day (in particular of preformed vitamin A) for up to 6 years. For example, >10,000 IU to 25,000 IU vitamin A per day (in particular of preformed vitamin A) for up to 6 years

Optionally the subject is administered up to 50% (for example >10% to 50%, or 25% to 50%) of a maximum safe dose of vitamin A (in particular of preformed vitamin A) for the subject per day.

For example, a maximum safe dose of vitamin A (in particular of preformed vitamin A) per day for an adult human subject may be 100,000 IU vitamin A (in particular of preformed vitamin A). Vitamin A toxicity levels have been recorded at around 1,000 IU/kg body weight (BW)/day for a horse. Thus, a maximum safe dose of vitamin A (in particular of preformed vitamin A) per day for an adult horse subject may be 1,000 IU/kg BW vitamin A (in particular of preformed vitamin A).

Optionally for a horse subject, the subject may be administered over 1,000,000 IU vitamin A per day, for example 1,500,000 to 2,000,000 IU vitamin per day.

The vitamin A may be administered to a subject systemically for example orally or intravenously.

Optionally vitamin A is used for inhibition of scar tissue formation in the subject resulting from an injury to the subject. The injury may be any injury that causes tissue damage. The injury may be a soft tissue injury which causes damage to soft tissue, such as muscle, a ligament, or a tendon. Such injuries may be caused, for example, by a strain, sprain, contusion, or a burn. The injury may be a neurological injury which causes damage to neurological tissue, such as a spinal cord injury, a brain injury, or a peripheral nerve injury.

The injury may be a traumatic injury. A traumatic injury is a physical injury of sudden onset and severity which requires immediate medical attention.

Where an injury involves a neurological injury, such as a spinal cord injury or a brain injury, vitamin A may be used to inhibit glial scar tissue formation.

Treatment with vitamin A may begin before surgery to repair the injured tissue.

Vitamin A treatment may begin after surgery to repair the injured tissue.

Vitamin A treatment may begin before surgery and be continued after surgery to repair the injured tissue.

Inhibition of scar tissue formation in accordance with the disclosure facilities regeneration of normal tissue, in particular by stem cells and/or quiescent cells present within or near a damaged tissue. Such stem cells and quiescent cells may be endogenous to the subject.

Quiescence is the reversible state of a cell in which it does not divide but retains the ability to re-enter cell proliferation. Some adult stem cells are maintained in a quiescent state and can be rapidly activated when stimulated, for example by damage or injury to the tissue in which they reside.

In some embodiments of the disclosure, one or more regenerative cells may be administered to the subject, for example by injection or transplantation.

Optionally vitamin A is administered to the subject prior to, with, or subsequent to administration of one or more regenerative cells.

There is also provided according to the disclosure vitamin A for use in inhibition of scar tissue formation in a subject, wherein the vitamin A is to be administered before, with, or after administration of one or more regenerative cells.

There is also provided according to the disclosure use of vitamin A in the manufacture of a medicament for the inhibition of scar tissue formation in a subject, wherein the vitamin A is to be administered before, with, or after administration of one or more regenerative cells.

There is also provided according to the disclosure vitamin A for use in the inhibition of scar tissue formation in a subject that has been administered one or more regenerative cells.

There is also provided according to the disclosure one or more regenerative cells for use in the inhibition of scar tissue formation in a subject that has been administered vitamin A.

There is also provided according to the disclosure use of vitamin A in the manufacture of a medicament for the inhibition of scar tissue formation in a subject that has been administered one or more regenerative cells.

There is also provided according to the disclosure use of one or more regenerative cells in the manufacture of a medicament for the inhibition of scar tissue formation in a subject that has been administered vitamin A.

There is also provided according to the disclosure a method of inhibiting scar tissue formation in a subject, which comprises administering an effective amount of vitamin A and one or more regenerative cells to the subject.

According to the disclosure there is provided vitamin A and one or more regenerative cells for use in the treatment of an injury to a subject.

There is also provided according to the disclosure use of vitamin A and one or more regenerative cells in the manufacture of a medicament for the treatment of an injury to a subject.

There is also provided according to the disclosure vitamin A for use in the treatment of an injury to a subject, wherein the vitamin A is to be administered before, with, or after administration of one or more regenerative cells.

There is also provided according to the disclosure use of vitamin A in the manufacture of a medicament for the treatment of an injury to a subject, wherein the vitamin A is to be administered before, with, or after administration of one or more regenerative cells.

There is also provided according to the disclosure vitamin A for use in the treatment of an injury to a subject that has been administered one or more regenerative cells.

There is also provided according to the disclosure one or more regenerative cells for use in the treatment of an injury to a subject that has been administered vitamin A.

There is also provided according to the disclosure use of vitamin A in the manufacture of a medicament for the treatment of an injury to a subject that has been administered one or more regenerative cells.

There is also provided according to the disclosure use of one or more regenerative cells in the manufacture of a medicament for the treatment of an injury to a subject that has been administered vitamin A.

There is also provided according to the disclosure a method of treating an injury to a subject, which comprises administering an effective amount of vitamin A and one or more regenerative cells to the subject.

According to the disclosure there is provided vitamin A and one or more regenerative cells for use in the treatment of tissue damage in a subject.

There is also provided according to the disclosure use of vitamin A and one or more regenerative cells in the manufacture of a medicament for the treatment of tissue damage in a subject.

There is also provided according to the disclosure vitamin A for use in the treatment of tissue damage in a subject, wherein the vitamin A is to be administered before, with, or after administration of one or more regenerative cells.

There is also provided according to the disclosure use of vitamin A in the manufacture of a medicament for the treatment of tissue damage in a subject, wherein the vitamin A is to be administered before, with, or after administration of one or more regenerative cells.

There is also provided according to the disclosure vitamin A for use in the treatment of tissue damage in a subject that has been administered one or more regenerative cells.

There is also provided according to the disclosure one or more regenerative cells for use in the treatment of tissue damage in a subject that has been administered vitamin A.

There is also provided according to the disclosure use of vitamin A in the manufacture of a medicament for the treatment of tissue damage in a subject that has been administered one or more regenerative cells.

There is also provided according to the disclosure use of one or more regenerative cells in the manufacture of a medicament for the treatment of tissue damage in a subject that has been administered vitamin A.

There is also provided according to the disclosure a method of treating tissue damage in a subject, which comprises administering an effective amount of vitamin A and one or more regenerative cells to the subject.

The tissue damage is tissue damage resulting from an injury to the subject.

The vitamin A and the one or more regenerative cells may be sourced together or separately.

A regenerative cell is a cell that has capacity to restore at least some functionality to an injured tissue. Examples of regenerative cells include stem cells, progenitor cells, mature cells, and *in vitro* or *ex vivo* differentiated stem cells.

Stem cells are cells that can differentiate into other types of cells, and can also divide in self-renewal to produce more of the same type of stem cells. In mammals, there are two broad types of stem cells: embryonic stem cells, which are isolated from the inner cell mass of blastocysts in early embryonic development, and adult stem cells, which are found in various tissues of fully developed mammals.

A progenitor cell is a cell that, like a stem cell, has a tendency to differentiate into a specific type of cell, but is already more specific than a stem cell and is pushed to differentiate into its "target" cell. The most important difference between stem cells and progenitor cells is that stem cells can replicate indefinitely, whereas progenitor cells can divide only a limited number of times. Most progenitors are described as oligopotent. In this point of view, they may be compared to adult stem cells. But progenitors are said to be in a further stage of cell differentiation. They are in the "centre" between stem cells and fully differentiated cells. Progenitors can go through several rounds of cell division before finally differentiating into a mature cell.

| | **Stem Cell** | **Progenitor Cell** |
|---|---|---|
| **Self-renewal in vitro** | Unlimited | Limited |
| **Potentiality** | Multipotent | Unipotent, sometimes oligopotent |
| **Maintenance of self-renewal** | Yes | No |
| **Population** | Reaches maximum number of cells before differentiating | Does not reach maximum population |

In adult organisms, stem cells and progenitor cells replenish adult tissues. In a developing embryo, stem cells can differentiate into all the specialized cells-ectoderm, endoderm and mesoderm but also maintain the normal turnover of regenerative organs, such as blood, skin, or intestinal tissues.

Examples of suitable stem cells include pluripotent stem cells (such as embryonic stem cells or induced pluripotent stem cells), multipotent stem cells, including multipotent stem cells capable of differentiating into neural cells (such as neural stem cells), and multipotent stem cells capable of differentiating into mesenchymal cells (such as mesenchymal stem cells, adipose-derived stem cells or tendon-derived stem cells).

Examples of suitable *in vitro* or *ex vivo* differentiated stem cells include *in vitro* or *ex vivo* differentiated pluripotent stem cells or *in vitro* or *ex vivo* differentiated multipotent stem cells.

Examples of suitable mature cells include neurons, glia and tenocytes.

Vitamin A may be administered before, with, or subsequent to administration of the one or more regenerative cells.

Administration of vitamin A with the one or more regenerative cells may be by coadministration of vitamin A with the one or more regenerative cells, or by simultaneous administration of vitamin A with the one or more regenerative cells (i.e. by separate administration at the same time, for example by different routes of administration).

Vitamin A may be administered to the subject before administration of the one or more regenerative cells. For example, the one or more regenerative cells may be administered within 6, 12, 24, 48, 72, or 96 hours after administration of vitamin A.

In other embodiments, vitamin A may be administered to the subject after administration of the one or more regenerative cells. For example, vitamin A may be administered within 6, 12, 24, 48, 72, or 96 hours of administration of the one or more regenerative cells.

It will be appreciated that vitamin A should preferably be administered as soon as possible after an injury has occurred. Optionally vitamin A is administered within a month, within a week, or within a day of the injury. Optionally vitamin A is administered with a week of the injury.

However, beneficial effects of treatment with vitamin A have also been observed when treatment is initiated many months or even years after an injury has occurred. Thus, optionally vitamin A may be administered months, years or even decades after an injury has occurred, for example within six months, a year, or a decade, or within twenty, thirty, forty, fifty, or sixty years of the injury.

The one or more regenerative cells may comprise one or more autologous cells. For example, a stem cell, progenitor, or mature cell may be taken from a subject, and optionally expanded and/or differentiated *in vitro* or *ex vivo,* before being administered back into the subject to aid restoration of at least some functionality to an injured tissue.

There are three known accessible sources of autologous adult stem cells in humans: bone marrow, adipose tissue, and blood. Adult stem cells may also be isolated from dental pulp, skin and most major organs and tissues of the body.

The one or more regenerative cells may comprise one or more allogeneic cells. For example, a stem cell or a mature cell may be taken from a healthy subject, and optionally expanded and/or differentiated *in vitro* or *ex vivo,* before being administered to the subject in need thereof to aid restoration of at least some functionality to an injured tissue.

In some cases, the one or more regenerative cells comprises one or more stem cells. Stem cells can be totipotent (i.e. they can differentiate into all the cell types in a body as well as the extraembryonic, or placental, cells); pluripotent (i.e. they can differentiate into all the cell types in a body); multipotent (i.e. they can differentiate into more than one cell type, but are more limited that pluripotent stem cells); or unipotent (i.e. they can differentiate into only one cell type).

Examples of pluripotent stem cells are embryonic stem cells (ESCs) which can be derived from the inner cell mass of embryos, including by parthogenesis, and induced pluripotent stem cells (iPSCs). Mature adult cells may be directed to a pluripotent state by addition of specific factors to the cells *in vitro.* These resulting cells are termed iPSCs. An iPSC can differentiate into all cell types in a body.

Therefore, in some cases the one or more stem cells of the disclosure may comprise one or more pluripotent stem cells such as one or more ESC or iPSC.

It may be advantageous to use multipotent stem cells for regenerative purposes. Mesenchymal stem cells (MSCs) are a type of multipotent stem cell found in large numbers in adults that are relatively easily isolated from bone marrow, adipose tissue and peri-vascular niches. MSCs and other multipotent stem cells such as tendon-derived stem cells may be particularly advantageous for restoring function to injured tendons. However, the differentiation potential of multipotent stem cells is more restricted than that of pluripotent stem cells.

In some cases the one or more stem cells may comprise one or more multipotent stem cells capable of differentiating into one or more neural cells, for example a neural stem cell. The neural cells may include neurons and/or glia.

In some cases the one or more stem cells may comprise one or more multipotent stem cells capable of differentiating into one or more mesenchymal cells, for example a mesenchymal stem cell, adipose-derived stem cell or tendon-derived stem cell. The mesenchymal cell may include tenocytes.

There are several different methods to differentiate stem cells to specific cell lineages. For example, a stem cell may be cultured *in vitro* or *ex vivo* in the presence of specific growth factors and/or morphogenic factors and/or small molecules (collectively termed 'differentiation factors') for specific times to direct differentiation to a specific cell lineage. The specific differentiation factor or combination thereof and the specific time of exposure to said factors will depend on the identity of the starting stem cell and the specific cell lineage required.

Mechanical factors may also be used control the ability of stem cells to differentiate towards specific lineages. For example, a substrate with a specific stiffness may be selected to control stem cell differentiation. Substrates with varying surface chemistry (for example hydrophobic/hydrophilic properties) and topography (for example the degree of folding of a particular substrate) may be selected to control stem cell differentiation.

Stem cells may also be differentiated to specific cell lineages by culturing the stem cells on or within naturally-derived biomaterials for example 3D collagen gels or decellularised scaffolds isolated from an *in vivo* microenvironment.

Differentiation protocols towards specific cell lineages for specific starting stem cells are well known to the person skilled in the art and may use a combination of the approaches outlined above. For example, differentiation of stem cells for tendon and spinal cord regeneration is reviewed in Lui Stem cell technology for tendon regeneration: current status, challenges, and future research directions Stem Cells and Cloning: Advances and Applications 2015:8 163-174 and briefly reviewed in Gazdic et al., Stem Cells Therapy for Spinal Cord Injury, Int. J. Mol. Sci. 2018, 19, 1039.

The one or more regenerative cells may comprise one or more *in vitro-* or *ex vivo-* differentiated stem cells for example neurons, glia, and/or tenocytes. In some cases, stem cells may be cultured and maintained as stem cells *in vitro* or *ex vivo* for example, to increase the number of stem cells compared to the starting number of stem cells, before being administered as stem cells into the subject in need thereof. Stem cells may be isolated from a heathy subject or tissue and stored before being administered to a subject in need thereof. Stem cells may be isolated from a healthy subject or tissue and optionally sorted, for example using flow-assisted cell sorting, before being immediately administered to a subject in need thereof.

The one or more regenerative cells may comprise one or more mature cells for example neurons, glia, and/or tenocytes.

Regenerative cells may be isolated from a heathy subject or tissue and stored before being administered to a subject in need thereof. Regenerative cells may be isolated from a healthy subject or tissue and optionally sorted, for example using flow-assisted cell sorting, before being immediately administered to a subject in need thereof. Regenerative cells may be cultured *in vitro* or *ex vivo* for example to increase the number of regenerative cells compared with the starting number of regenerative cells and/or to differentiate the regenerative cells to specific cell lineages (such as neurons, glia or tenocytes).

The one or more regenerative cells may be administered locally i.e. local to a site of injury that is in need of repair. For example, a regenerative cell may be injected or transplanted into a tendon in need of repair and/or into one or more lesions of a tendon in need of repair; a regenerative cell may be administered by a direct intramedullary or intrathecal injection or transplantation of a spinal cord in need of repair and/or into one or more lesions of a spinal cord in need of repair. Alternatively, a regenerative cell may be administered distally i.e. distal to a site of injury that is in need of repair. For example a regenerative cell may be injected intravenously and/or intraperitoneally and/or subcutaneously for tendon and/or spinal cord repair. Alternatively, regenerative cells may be administered both locally and distally, or locally and systemically, or distally and systemically, or locally and distally and systemically. Routes of administration for regenerative cells have been reviewed in Lui Stem cell technology for tendon regeneration: current status, challenges, and future research directions Stem Cells and Cloning: Advances and Applications 2015:8 163-174 and Oh et al. Current Concept of Stem Cell Therapy for Spinal Cord Injury: A Review 2016; 12(2):40-46*.*

Regenerative cells may be administered to a subject in need thereof at the same time as surgery takes place to repair the injured tissue. Regenerative cells may be administered to a subject in need thereof after surgery to repair the injured tissue.

Regenerative cells may be administered to a subject in need thereof multiple times. Each regenerative cell administration subsequent to the initial regenerative cell administration may be termed a "top up". Each top up may be administered via the same or a different route as the initial administration. Thus a top up may be administered locally. Alternatively, a top up may be administered distally.

The top up administration may be up to one year later than the initial regenerative cell administration. For example, the top up administration may be up to six months later than the initial regenerative cell administration; up to three months later than the initial regenerative cell administration; up to one month later than the initial regenerative cell administration; or up to one week later than the initial regenerative cell administration.

As regenerative cells may be administered to a subject in need thereof multiple times, each top up administration may take place at regular time intervals. For example, each top up administration may take place weekly, monthly, quarterly, bi-annually, or annually.

Each top up administration may be with any regenerative cell. For example, each top up administration may be with the same regenerative cell as the initial regenerative cell administered. Alternatively, each top up administration may be with a different regenerative cell to the initial regenerative cell administered.

Also provided according to the invention is a multiple-dose formulation for use in the treatment of tissue damage in a human subject resulting from an injury to the subject, wherein the injury comprises a soft tissue injury, wherein the soft tissue injury is a tendon injury or a ligament injury, wherein the multiple-dose formulation comprises a plurality of separate unit doses of vitamin A, wherein each unit dose comprises >10,000 IU to 100,000 IU vitamin A.

Optionally, the multiple-dose formulation comprises a plurality of unit doses of vitamin A wherein each unit dose comprises up to 100,000 IU vitamin A, for example >10,000 IU to 100,000 IU vitamin A; 25,000 to 100,000 IU vitamin A; 50,000 to 100,000 IU vitamin A; or 75,000 to 100,000 IU vitamin A (in particular of preformed vitamin A).

Vitamin A of a multiple-dose formulation of the invention may comprise any combination of vitamin A described previously.

A multiple-dose formulation of the invention may comprise at least 7 unit doses, at least 30 unit doses, or at least 100 unit doses of vitamin A.

Each unit dose of vitamin A in a multiple-dose formulation of the invention may comprise a pharmaceutical composition comprising vitamin A and a pharmaceutically acceptable carrier, excipient or diluent.

There is also provided according to the disclosure a multiple-dose formulation for use in inhibition of scar tissue formation in a subject.

Vitamin A and the one or more regenerative cells may be provided as a combined preparation.

According to the disclosure there is provided a combined preparation comprising: (a) vitamin A; and (b) one or more regenerative cells.

The term "combined preparation" as used herein refers to a "kit of parts" in the sense that the combination components (a) and (b) as defined above can be dosed independently or by use of different fixed combinations with distinguished amounts of the combination components (a) and (b). The components can be administered simultaneously or one after the other. If the components are administered one after the other, preferably the time interval between administration is chosen such that the therapeutic effect of the combined use of the components is greater than the effect which would be obtained by use of only any one of the combination components (a) and (b).

The components of the combined preparation may be present in one combined unit dosage form, or as a first unit dosage form of component (a) and a separate, second unit dosage form of component (b). The ratio of the total amounts of the combination component (a) to the combination component (b) to be administered in the combined preparation can be varied, for example in order to cope with the needs of a single patient, which can be due, for example, to the particular condition, age, sex, or body weight of the patient.

A combined preparation of the disclosure may be provided as a pharmaceutical combined preparation for administration to human, or a horse. The vitamin A may optionally be provided together with a pharmaceutically acceptable carrier, excipient, or diluent, and/or the one or more regenerative cells may optionally be provided together with a pharmaceutically acceptable carrier, excipient, or diluent.

The combined preparation may comprise up to 100,000 IU vitamin A. The combined preparation may comprise any combination of vitamin A described previously.

Use of vitamin A, optionally with one or more regenerative cells, in accordance with the invention may be particularly effective for the treatment of older subjects. For example, a human subject may be at least 18 years old, at least 25 years old, at least 30 years old, at least 40 years old, or at least 50 years old.

Uses and methods of the disclosure may be particularly advantageous for the treatment of tendon injury in a horse. According to an aspect which is not covered by the present invention the uses and methods of the disclosure may be advantageous for the treatment of a neurological injury (for example, a spinal cord injury) in a dog.

The vitamin A and/or the one or more regenerative cells can be incorporated into a variety of formulations for therapeutic administration, more particularly by combination with appropriate, pharmaceutically acceptable carriers, pharmaceutically acceptable diluents, or other pharmaceutically acceptable excipients, and can be formulated into preparations in solid, semi-solid, liquid or gaseous forms, such as tablets, capsules, powders, granules, ointments, solutions, suppositories, injections, inhalants and aerosols as appropriate.

Optionally the vitamin A is in solid form.

Optionally the vitamin A is not in an organic solution.

Optionally the vitamin A is not encapsulated by, or attached to a microparticle.

Optionally the vitamin A is not encapsulated by, or attached to a nanoparticle.

Vitamin A can be administered in the form of a pharmaceutically acceptable salt. It can also be used alone or in appropriate association, as well as in combination, with other pharmaceutically active compounds. Optionally vitamin A is administered with an antibiotic agent. Optionally vitamin A is the only non-cellular, non-antibiotic, active agent administered.

The following methods and excipients are merely exemplary and are in no way limiting.

For oral preparations, vitamin A can be used alone or in combination with appropriate additives to make tablets, powders, granules or capsules, for example, with conventional additives, such as lactose, mannitol, corn starch or potato starch; with binders, such as crystalline cellulose, cellulose derivatives, acacia, corn starch or gelatins; with disintegrators, such as corn starch, potato starch or sodium carboxymethylcellulose; with lubricants, such as talc or magnesium stearate; and if desired, with diluents, buffering agents, moistening agents, preservatives and flavoring agents.

Vitamin A and/or the one or more regenerative cells can be formulated into preparations for injection by dissolving, suspending or emulsifying in an aqueous or nonaqueous solvent, such as vegetable or other similar oils, propylene glycol, synthetic aliphatic acid glycerides, injectable organic esters (e.g., ethyl oleate), esters of higher aliphatic acids or propylene glycol; and if desired, with conventional additives such as solubilizers, isotonic agents, suspending agents, emulsifying agents, stabilizers and preservatives. Parenteral vehicles include sodium chloride solution, Ringer's dextrose, dextrose and sodium chloride, lactated Ringer's, or fixed oils. Intravenous vehicles include fluid and nutrient replenishers, electrolyte replenishers (such as those based on Ringer's dextrose), and the like. Furthermore, a pharmaceutical composition of the present disclosure can comprise further agents such as dopamine or psychopharmacologic drugs, depending on the intended use of the pharmaceutical composition.

Pharmaceutical compositions are prepared by mixing Vitamin A having the desired degree of purity, and/or the one or more regenerative cells with optional physiologically acceptable carriers, other excipients, stabilizers, surfactants, buffers and/or tonicity agents. Acceptable carriers, other excipients and/or stabilizers are nontoxic to recipients at the dosages and concentrations employed, and may include buffers such as phosphate, citrate, and other organic acids; antioxidants including ascorbic acid, glutathione, cysteine, methionine and citric acid; preservatives (such as ethanol, benzyl alcohol, phenol, m-cresol, p-chlor-m-cresol, methyl or propyl parabens, benzalkonium chloride, or combinations thereof); amino acids such as arginine, glycine, ornithine, lysine, histidine, glutamic acid, aspartic acid, isoleucine, leucine, alanine, phenylalanine, tyrosine, tryptophan, methionine, serine, proline and combinations thereof; monosaccharides, disaccharides and other carbohydrates; low molecular weight (less than about 10 residues) polypeptides; proteins, such as gelatin or serum albumin; chelating agents such as EDTA; sugars such as trehalose, sucrose, lactose, glucose, mannose, maltose, galactose, fructose, sorbose, raffinose, glucosamine, N-methylglucosamine, galactosamine, and neuraminic acid; and/or non-ionic surfactants such as Tween, Brij Pluronics, Triton-X, or polyethylene glycol (PEG).

The pharmaceutical composition can be in a liquid form, a lyophilized form or a liquid form reconstituted from a lyophilized form, wherein the lyophilized preparation is to be reconstituted with a sterile solution prior to administration. The standard procedure for reconstituting a lyophilized composition is to add back a volume of pure water (typically equivalent to the volume removed during lyophilization); however solutions comprising antibacterial agents can be used for the production of pharmaceutical compositions for parenteral administration; see also Chen (1992) Drug Dev Ind Pharm 18, 1311-54.

An aqueous formulation can be prepared in a pH-buffered solution, e.g., at pH ranging from about 4.0 to about 7.0, or from about 5.0 to about 6.0, or alternatively about 5.5. Examples of buffers that are suitable for a pH within this range include phosphate-, histidine-, citrate-, succinate-, acetate-buffers and other organic acid buffers. The buffer concentration can be from about 1 mM to about 100 mM, or from about 5 mM to about 50 mM, depending, e.g., on the buffer and the desired tonicity of the formulation.

A tonicity agent can be included in the formulation to modulate the tonicity of the formulation. Exemplary tonicity agents include sodium chloride, potassium chloride, glycerin and any component from the group of amino acids, sugars as well as combinations thereof. In some embodiments, the aqueous formulation is isotonic, although hypertonic or hypotonic solutions can be suitable. The term "isotonic" denotes a solution having the same tonicity as some other solution with which it is compared, such as a physiological salt solution or serum. Tonicity agents can be used in an amount of about 5 mM to about 350 mM, e.g., in an amount of 100 mM to 350 nM.

A surfactant can also be added to the formulation to reduce aggregation and/or minimize the formation of particulates in the formulation and/or reduce adsorption. Exemplary surfactants include polyoxyethylensorbitan fatty acid esters (Tween), polyoxyethylene alkyl ethers (Brij), alkylphenylpolyoxyethylene ethers (Triton-X), polyoxyethylene-polyoxypropylene copolymer (Poloxamer, Pluronic), and sodium dodecyl sulfate (SDS). Examples of suitable polyoxyethylenesorbitan-fatty acid esters are polysorbate 20, (sold under the trademark Tween 20^{™}) and polysorbate 80 (sold under the trademark Tween 80^{™}). Examples of suitable polyethylene-polypropylene copolymers are those sold under the names Pluronic^{®} F68 or Poloxamer 188^{™}. Examples of suitable Polyoxyethylene alkyl ethers are those sold under the trademark Brij^{™}. Exemplary concentrations of surfactant can range from about 0.001% to about 1% w/v.

A lyoprotectant can also be added in order to protect a labile active ingredient against destabilizing conditions during the lyophilization process. For example, known lyoprotectants include sugars (including glucose and sucrose); polyols (including mannitol, sorbitol and glycerol); and amino acids (including alanine, glycine and glutamic acid). Lyoprotectants can be included in an amount of about 10 mM to 500 nM.

In some embodiments, a subject formulation includes one or more of the above-identified agents (e.g., a surfactant, a buffer, a stabilizer, a tonicity agent) and is essentially free of one or more preservatives, such as ethanol, benzyl alcohol, phenol, m-cresol, p-chlor-m-cresol, methyl or propyl parabens, benzalkonium chloride, and combinations thereof. In other embodiments, a preservative is included in the formulation, e.g., at concentrations ranging from about 0.001 to about 2% (w/v).

Unit dosage forms for oral administration such as syrups, elixirs, and suspensions can be provided wherein each dosage unit, for example, teaspoonful, tablespoonful, or tablet contains a predetermined amount of the active agent (i.e. vitamin A and/or the one or more regenerative cells). Similarly, unit dosage forms for injection or intravenous administration can comprise vitamin A and/or the one or more regenerative cells in a composition as a solution in sterile water, normal saline or another pharmaceutically acceptable carrier.

The term "unit dosage form," as used herein, refers to physically discrete units suitable as unitary dosages for human and animal subjects, each unit containing a predetermined quantity of vitamin A and/or the one or more regenerative cells, calculated in an amount sufficient to produce the desired effect in association with a pharmaceutically acceptable diluent, carrier or vehicle.

Vitamin A and/or the one or more regenerative cells can be administered as an injectable formulation. Typically, injectable compositions are prepared as liquid solutions or suspensions; solid forms suitable for solution in, or suspension in, liquid vehicles prior to injection can also be prepared.

Suitable excipient vehicles are, for example, water, saline, dextrose, glycerol, ethanol, or the like, and combinations thereof. In addition, if desired, the vehicle can contain minor amounts of auxiliary substances such as wetting or emulsifying agents or pH buffering agents. Actual methods of preparing such dosage forms are known, or will be apparent, to those skilled in the art. See, e.g., Remington's Pharmaceutical Sciences, Mack Publishing Company, Easton, Pennsylvania, 17th edition, 1985. The composition or formulation to be administered will, in any event, contain a quantity of vitamin A and/or the one or more regenerative cells adequate to achieve the desired state in the subject being treated.

The pharmaceutically acceptable excipients, such as vehicles, adjuvants, carriers or diluents, are readily available to the public. Moreover, pharmaceutically acceptable auxiliary substances, such as pH adjusting and buffering agents, tonicity adjusting agents, stabilizers, wetting agents and the like, are readily available to the public.

Ranges may be expressed herein as from "about" one particular value, and/or to another particular value. When such a range is expressed, another embodiment includes from the one particular value and/or to the other particular value. Similarly, when values are expressed as approximations, by the use of the antecedent "about", it will be understood that the particular value forms another embodiment.

Wherever the term "vitamin A" is used herein this includes reference to "vitamin A or a pharmaceutically acceptable salt thereof".

Optionally uses and methods of the disclosure are limited to cosmetic treatment of a subject.

Optionally uses and methods of the disclosure are for non-cosmetic treatment of a subject.

Methods for visualising scar tissue formation are known to those of ordinary skill in the art. Examples include MRI for spinal cord, and tendons, and ultrasound in tendons. Suitable examples are described in the following documents:
MRI in spinal cord injury:
   - Ellingson et al., "Imaging Techniques in Spinal Cord Injury", World Neurosurg. 2014 Dec; 82(6): 1351-1358;
   - Hu et al., "Glial scar and neuroregeneration: histological, functional, and magnetic resonance imaging analysis in chronic spinal cord injury", Journal of Neurosurgery, 2010, 13(2) (doi.org/10.3171/2010.3.SPINE09190);
   - Byrnes et al., "Neuropathological Differences Between Rats and Mice after Spinal Cord Injury", J Magn Reson Imaging. 2010 Oct; 32(4): 836-846.
MRI in tendon injury:
   - Galatz et al., "Tendon Regeneration and Scar Formation: The Concept of Scarless Healing", Journal Of Orthopaedic Research, 2015, 823-831;
   - Tavares Jr, et al., "Healing of the Achilles tendon in rabbits-evaluation by magnetic resonance imaging and histopathology", Journal of Orthopaedic Surgery and Research, volume 9, Article number: 132 (2014);
Ultrasound in tendon injury:
   - Ackerman, et al., "Non-Invasive Ultrasound Quantification of Scar Tissue Volume Identifies Early Functional Changes During Tendon Healing", Volume 37, Issue 11, November 2019, Pages 2476-2485;
   - Galatz et al., "Tendon Regeneration and Scar Formation: The Concept of Scarless Healing", Journal Of Orthopaedic Research, 2015, 823-831

Embodiments of the invention are described below, by way of example only.

### Example 1

### Tendon Injury - recovery following treatment with Vitamin A

| | |
|---|---|
| **Patient** | Horse subject 1 (polo pony) |
| **Age** | 14 years |
| **Sex** | Mare |
| **Health status** | Good |

### Injury

| | |
|---|---|
| **Type of injury** | Tendon injury |
| **When the injury occurred** | 14 February 2019 |
| **Cause of the injury** | Hyperextension and/or blunt trauma |
| **Tissue(s) affected** | Tendon |
| **Impairments resulting from the injury** | Lameness and loss of athletic function |

### Treatment with conventional methods

Single dose 30mg dexamethasone IV to treat the initial acute inflammation followed by 1g phenylbutazone PO BID for 5 days. Ice applied to affected area for 15 minutes twice a day for 5 days.

### Extent of recovery following treatment with conventional methods

Recovery has progressed as expected with these types of lesions in horses. Moderate lameness, heat and pain response to firm digital palpation improved rapidly over the first 7 to 10 days. The horse then started a programme of incremental walking exercise.

### Treatment with Vitamin A

**How soon after the injury was treatment was treatment with Vitamin A initiated?** Treatment with Vitamin A was initiated approximately 20 days after the initial injury.
**Type of Vitamin A administered**
   Retinyl palmitate (93.8% Retinol Equivalent)
**Route of administration**
   Oral
**Dose of Vitamin A per administration**
   750,000 IU
**Frequency of administration**
   Twice daily (1,500,000 IU per day)
**Period of administration**
   Continuing (at least 4 weeks)

### Recovery following treatment with Vitamin A

Functional recovery has been good considering the severity of the lesion. Ultrasonography has confirmed good reduction in the size of the lesion (notable reduction in size of anechoic pockets of interstitial haemorrhage). Horses are prey species and often do not show signs of overt pain or lameness even with severe lesions. It is not uncommon for such lesions to take between 12 to 18 months to heal.

### Example 2: Patient-reported outcome measure

### Spinal cord Injury - recovery following treatment with Vitamin A

| | |
|---|---|
| **Patient** | Human Subject 1 |
| **Age** | 68 |
| **Sex** | Male |
| **Health status** | Good |

### Injury

| | |
|---|---|
| **Type of injury** | Spinal cord injury |
| **When the injury occurred** | November, 1963 |
| **Cause of the injury** | Trauma to T4 |
| **Tissue(s) affected** | Left lateral spinothalamic tract (Brown-Sequard syndrome) |
| **Impairments resulting from the injury** | Left stiff knee, right lower back pain, difficulty walking |

### Treatment with conventional methods

Laminectomy T4 and T5
Baclofen 20 mg AM and 30 mg PM for the past 13 years.

### Extent of recovery following treatment with conventional methods

Seven years after my operation (1963) I was very active and had a Brown Belt in Tae Kwon Do, I started to limp when I was 24 years old. At 38 years I could not run. At 52 my limp was more pronounced and I had a series of Botox injections to my left leg and thigh muscles over a period of 2-3 years. At 55 I was started on Baclofen and currently I take 20 mg every morning and 30 mg at night. At 64 my mobility continues to decrease and I began using a cane to ambulate. I began to experience pain of my right lower back approximately 4 years ago. It ranges from a scale of 2-6/10 depending on activities I do as well as the weather.

### Treatment with Vitamin A

**How soon after the injury was treatment was treatment with Vitamin A initiated?** 56 years
**Type of Vitamin A administered**
   Vitamin A 50,000 IU (from Retinyl palmitate and fish liver oil)
**Route of administration**
   Oral
**Dose of Vitamin A per administration**
   50,000 IU
**Frequency of administration**
   Once daily
**Period of administration**
   3 months

### Recovery following treatment with Vitamin A

The pain is approximately 10-15% less and when I have it, the duration is much less. The weather conditions have to be severe before I begin to experience pain. Previously I could forecast if it was going to rain a day or two ahead because my right lower back would start to ache. Now the weather conditions have to be more severe for my back to start aching.

### Example 3: Patient-reported outcome measure

### Spinal cord Injury - recovery following treatment with Vitamin A

| | |
|---|---|
| **Patient** | Human Subject 2 |
| **Age** | 69 |
| **Sex** | Male |

### Injury

| | |
|---|---|
| **Type of injury** | Spinal cord injury |
| **When the injury occurred** | Beginning of September 2016 |
| **Cause of the injury** | Spinal stenosis |
| **Tissue(s) affected** | Paralyzed both legs and no feeling up to chest |
| **Impairments resulting from the injury** | Both legs paralyzed and up to the chest |

### Treatment with conventional methods

First surgery 22^{nd} of September 2016, T3 to T4 (Considerable bleeding occurred during the operation). Second surgery in two places (laminotoimiu) on 9^{th} of March 2017, Th10-Th12 and L1-L5.

Drugs: After surgery 2016: Paracetamol 1gx4, Codein 30mgx4, Valsartan 82,5x1, Atorvastatin 40mgx1. Omeprazol 20mgx1, D3 vitamin 2000 aex1, Kaleorid 750 mgx1, Klexane 40mgx1, Betolvex 1 mgx1, Valsartan/hydrochlortiazide 80/12,5 x1, Atacor 40mgx1, Gabapentin 300plus 900 mg, Zopiclone 7,5 mg vesp. Tradolan 50 mg 2x2,
2019: Losartan/Hydrochlorothiazide 100mg/25mg x1, Acetylaslisylsyre 75 mgx1, Diclomex Rapid 50 mg 1x3, Anti Leg Cramps (NAVEH) 2x1, Vitamin A10.000x5. Imovane 7,5 mg 1x1. Milk Thistle(Lamberts) 8500mg of seed-Providing Silymarin 200mg 1x1.

### Extent of recovery following treatment with conventional methods

Daily exercises from the beginning. Wheelchair and crutches. Cramps in legs, need to take Gapabentin 3-5 pr day and Tradolan 2 pr day.

### Treatment with Vitamin A

**How soon after the injury was treatment was treatment with Vitamin A initiated?** Starting in October 2018. For three months taking vitamin A 10,000 x 3. Increasing in end January 2019 10,000 x 5.
**Type of Vitamin A administered**
   Vitamin A (from cod liver oil and vitamin A palmitate)
**Route of administration**
   Oral
**Dose of Vitamin A per administration**
   Initially total of 30,000 IU; From end of January 2019 total of 50,000 IU; 3,000 mcg RAE
**Frequency of administration**
   For 3 months 3 tablets a day and for 3 months 5 tablets a day
**Period of administration**
   6 months

### Recovery following treatment with Vitamin A

First I found recovery more walking control and balance. Coordination was better. In mid-January 2019 a big pain in left leg and ankle and could not train my left legs. My doctor said it was probably because of one of the pockets with water drops managed to get into my spinal cord. I asked him if that could happen again, he said no, may be only 10% possibility. This problem took me 1.5 months to recover. Now in March 2019 I feel much stronger, more balanced, more movement control and I am walking as a whole 6x42 steps (up and down) twice in a week. In beginning of April start walking without any support on even level 30-50 meters with better controlling.

Subject provided videos showing the subject walking along an even level with very little to no support and walking up and down a set of stairs.

### Example 4: Patient-reported outcome measure

### Spinal cord Injury - recovery following treatment with Vitamin A

| | |
|---|---|
| **Patient** | Human Subject 3 |
| **Age** | 79 |
| **Sex** | Male |
| **Health status** | Good |

### Injury

| **Type of injury** | Disc/nerve pain |
|---|---|
| **When the injury occurred** | 2018 |
| **Tissue(s) affected** | Facet joint |
| **Impairments resulting from the injury** | Pain around knee (limping) |

Treatment with conventional methods
   Injections to disc/nerve x 2
Treatment with Vitamin A
   **How soon after the injury was treatment with Vitamin A initiated?**
   3/4 months
**Type of Vitamin A administered**
   Vitamin A 50,000 IU
**Route of administration**
   Oral
**Dose of Vitamin A per administration**
   50,000:1
**Frequency of administration**
   Daily
**Period of administration**
   4-5 months

### Recovery following treatment with Vitamin A

Vitamin A has improved pain level which has almost disappeared x 80%.

### Example 5: Patient-reported outcome measure

### Spinal cord Injury - recovery following treatment with Vitamin A

| **Patient** | Human Subject 4 |
|---|---|
| **Age** | 60 |
| **Sex** | Male |

### Injury

| | |
|---|---|
| **Type of injury** | Spinal cord injury and now have lumbar spinal stenosis as well |
| **When the injury occurred** | Spinal Cord Injury in March 1998 at C4/C5 level |
| **Cause of the injury** | Fell off mountain in France skiing |
| **Tissue(s) affected** | Incomplete injury, initial paralysis below C4/C5 but reasonable mobility has been recovered. All tissues effected below C4/C5 |
| **Impairments resulting from the injury** | Loss of some mobility below C4/C5 level, I walk with a stick. |

### Treatment with conventional methods

In 1998 I had a laminectomy after the accident in France, and then a disc fusion in London both at C4/C5 level.

In 2009 I had a further disc replacement in my neck after a fall.

I had injections in my neck in May 2018 for stenosis and severe neck pain.

I have had injections in my lumbar region in May and September 2018 and March 2019 and rhizolysis in the lumbar region in September 2018 and at 3 levels in March 2019.

I have intermittently taken pain killers including ibuprofen, paracetomol and naproxen. In February 2019 I took Arcoxia for 4 weeks. I also take antibiotics as a prophylaxis to prevent UTls. I also take toltoredine, topsium chloridfe, senna and diactyl.

### Extent of recovery following treatment with conventional methods

Good recovery from the original spinal cord injury in my neck but it left me with very impaired mobility and walking with a stick.

After the injection in my neck in May 2018 this significantly helped remove the pain but it left me with a very stiff neck which still ached quite a lot but the pain was bearable.

Improved mobility since the injection and rhizolysis in lumbar region in March 2019.

### Treatment with Vitamin A

**How soon after the injury was treatment was treatment with Vitamin A initiated?**
21 years later in February 2019
**Type of Vitamin A administered**
   Vitamin A (from cod liver oil and vitamin A palmitate)
**Route of administration**
   Oral
**Dose of Vitamin A per administration**
   Total of 50,000 IU (5 x 10,000 IU tablets)
**Frequency of administration**
   I take 50,000 IU in one administration in the morning daily
**Period of administration**
   Continuing: Almost 2 months

### Recovery following treatment with Vitamin A

Very soon after taking Vitamin A my neck was much more flexible and the aching largely disappeared. I have much more movement in my neck and the aching has now disappeared.

Big improvement in overall mobility, walking and feeling of better health following the rhizolysis in early March 2019 - I do not know if this all due to the procedure or whether this improvement is due to the Vitamin A. but the big improvement in my neck before the procedure was after taking Vitamin A and continued after I stopped taking the Arcoxia.

## Claims

1. Vitamin A for use in the treatment of tissue damage in a subject following an injury to the subject, wherein the injury comprises a soft tissue injury, wherein the soft tissue injury is a tendon injury or a ligament injury, wherein the vitamin A is to be administered systemically by oral or intravenous administration to the subject, and wherein:
a) the subject is a human subject and the vitamin A is for administration to the subject at a dose of >10,000 to 100,000 IU vitamin A per day; or
b) the subject is a horse and the vitamin A is for administration to the horse at a dose of 250 to 500 IU/kg body weight per day.

2. Vitamin A for use according to claim 1, wherein the subject is a human subject, and the vitamin A is for administration to the subject at a dose of 25,000-100,000, 50,000-100,000, or 75,000-100,000 IU vitamin A per day.

3. Vitamin A for use according to claim 1, wherein the subject is an adult human subject, and the vitamin A is for administration to the subject at a dose of >10,000 to 50,000 IU vitamin A per day, or 25,000 to 50,000 IU vitamin A per day.

4. Vitamin A for use according to claim 1 or 3, wherein the subject is a human subject, and the vitamin A is to be administered at >10% to 50%, or 25% to 50% of a maximum safe dose of vitamin A for the subject per day.

5. Vitamin A for use according to claim 1, wherein the subject is a horse, and the vitamin A is to be administered at 25% to 50% of a maximum safe dose of vitamin A for the subject per day.

6. Vitamin A for use according to any preceding claim, wherein the vitamin A comprises isolated vitamin A, a preformed vitamin A, such as a retinyl ester or retinol, a provitamin A, such as a carotenoid, or a bioactive form of vitamin A, such as retinal or retinoic acid.

7. Vitamin A for use according to any preceding claim for administration to the subject up to five times per day, for at least a week, at least a month, at least 6 months, or up to 6 years from the day of first administration to the subject.

8. Vitamin A for use according to any preceding claim, wherein the vitamin A is the only non-cellular, non-antibiotic, active agent to be administered to the subject.

9. A multiple-dose formulation for use in the treatment of tissue damage in a human subject resulting from an injury to the subject, wherein the injury comprises a soft tissue injury, wherein the soft tissue injury is a tendon injury or a ligament injury, wherein the multiple-dose formulation comprises a plurality of separate unit doses of vitamin A, wherein each unit dose comprises >10,000 IU to 100,000 IU vitamin A.

10. A multiple-dose formulation for use according to claim 9, wherein each unit dose comprises 25,000-100,000, 50,000-100,000, or 75,000-100,000 IU vitamin A.

11. A multiple-dose formulation for use according to claim 9 or 10, wherein the vitamin A comprises isolated vitamin A, a preformed vitamin A, such as a retinyl ester or retinol, a provitamin A, such as a carotenoid, or a bioactive form of vitamin A, such as retinal or retinoic acid.

12. A multiple-dose formulation for use according to any of claims 9 to 11, wherein the vitamin A is part of a pharmaceutical composition comprising vitamin A and a pharmaceutically acceptable carrier, excipient or diluent.

13. A multiple-dose formulation for use according to claim 12, wherein vitamin A is the only non-cellular, non-antibiotic, active agent present in the pharmaceutical composition.

14. A multiple-dose formulation for use according to any of claims 9 to 13 comprising at least 7, at least 30, or at least 100 separate unit doses of vitamin A.

15. A multiple-dose formulation for use according to any of claims 9 to 14, wherein the unit doses are for oral administration.

## Patentansprüche

1. Vitamin A zur Verwendung bei der Behandlung von Gewebeschäden in einem Subjekt nach einer Verletzung des Subjekts, wobei die Verletzung eine Weichgewebeverletzung umfasst, wobei die Weichgewebeverletzung eine Sehnenverletzung oder eine Bandverletzung ist, wobei das Vitamin A systemisch durch orale oder intravenöse Verabreichung an das Subjekt zu verabreichen ist, und wobei:
a) das Subjekt ein menschliches Subjekt ist und das Vitamin A zur Verabreichung an das Subjekt in einer Dosis von >10.000 bis 100.000 IU Vitamin A pro Tag bestimmt ist; oder
b) das Subjekt ein Pferd ist und das Vitamin A zur Verabreichung an das Pferd in einer Dosis von 250 bis 500 IU/kg Körpergewicht pro Tag bestimmt ist.

2. Vitamin A zur Verwendung nach Anspruch 1, wobei das Subjekt ein menschliches Subjekt ist und das Vitamin A zur Verabreichung an das Subjekt in einer Dosis von 25.000-100.000, 50.000-100.000 oder 75.000-100.000 IU Vitamin A pro Tag bestimmt ist.

3. Vitamin A zur Verwendung nach Anspruch 1, wobei das Subjekt ein erwachsenes menschliches Subjekt ist und das Vitamin A zur Verabreichung an das Subjekt in einer Dosis von >10.000 bis 50.000 IU Vitamin A pro Tag oder 25.000 bis 50.000 IU Vitamin A pro Tag bestimmt ist.

4. Vitamin A zur Verwendung nach Anspruch 1 oder 3, wobei das Subjekt ein menschliches Subjekt ist und das Vitamin A in einer Menge von >10 % bis 50 % oder 25 % bis 50 % einer maximalen sicheren Dosis von Vitamin A für das Subjekt pro Tag verabreicht werden soll.

5. Vitamin A zur Verwendung nach Anspruch 1, wobei das Subjekt ein Pferd ist und das Vitamin A in einer Menge von 25 % bis 50 % einer maximalen sicheren Dosis von Vitamin A für das Subjekt pro Tag verabreicht werden soll.

6. Vitamin A zur Verwendung nach einem der vorstehenden Ansprüche, wobei das Vitamin A isoliertes Vitamin A, ein vorgebildetes Vitamin A, wie einen Retinylester oder Retinol, ein Provitamin A, wie ein Carotinoid, oder eine bioaktive Form von Vitamin A, wie Retinal oder Retinsäure, umfasst.

7. Vitamin A zur Verwendung nach einem der vorstehenden Ansprüche zur Verabreichung an das Subjekt bis zu fünfmal pro Tag, für mindestens eine Woche, mindestens einen Monat, mindestens 6 Monate oder bis zu 6 Jahre ab dem Tag der ersten Verabreichung an das Subjekt.

8. Vitamin A zur Verwendung nach einem der vorstehenden Ansprüche, wobei das Vitamin A der einzige nicht-zelluläre, nicht-antibiotische Wirkstoff ist, der dem Patienten verabreicht wird.

9. Mehrfachdosisformulierung zur Verwendung bei der Behandlung von Gewebeschäden in einem menschlichen Subjekt, die aus einer Verletzung des Subjekts resultieren, wobei die Verletzung eine Weichgewebeverletzung umfasst, wobei die Weichgewebeverletzung eine Sehnenverletzung oder eine Bandverletzung ist, wobei die Mehrfachdosisformulierung eine Vielzahl von separaten Einheitsdosen von Vitamin A umfasst, wobei jede Einheitsdosis >10.000 IU bis 100.000 IU Vitamin A umfasst.

10. Mehrfachdosisformulierung zur Verwendung nach Anspruch 9, wobei jede Einheitsdosis 25.000-100.000, 50.000-100.000 oder 75.000-100.000 IU Vitamin A umfasst.

11. Mehrfachdosierungsformulierung zur Verwendung nach Anspruch 9 oder 10, wobei das Vitamin A isoliertes Vitamin A, ein vorgeformtes Vitamin A, wie einen Retinylester oder Retinol, ein Provitamin A, wie ein Carotinoid, oder eine bioaktive Form von Vitamin A, wie Retinal oder Retinsäure, umfasst.

12. Mehrfachdosierungsformulierung zur Verwendung nach einem der Ansprüche 9 bis 11, wobei das Vitamin A Teil einer pharmazeutischen Zusammensetzung ist, die Vitamin A und einen pharmazeutisch unbedenklichen Träger, Hilfsstoff oder Verdünnungsmittel umfasst.

13. Mehrfachdosierungsformulierung zur Verwendung nach Anspruch 12, wobei Vitamin A der einzige nicht-zelluläre, nicht-antibiotische Wirkstoff in der pharmazeutischen Zusammensetzung ist.

14. Mehrfachdosierungsformulierung zur Verwendung nach einem der Ansprüche 9 bis 13, umfassend mindestens 7, mindestens 30 oder mindestens 100 separate Einheitsdosen Vitamin A.

15. Mehrfachdosisformulierung zur Verwendung nach einem der Ansprüche 9 bis 14, wobei die Einheitsdosen zur oralen Verabreichung bestimmt sind.

## Revendications

1. Vitamine A pour utilisation dans le traitement d'une lésion tissulaire chez un sujet à la suite d'une blessure causée au sujet, dans laquelle la blessure comprend une blessure des tissus mous, dans laquelle la blessure des tissus mous est une blessure tendineuse ou une blessure ligamentaire, dans laquelle la vitamine A doit être administrée systémiquement par administration orale ou intraveineuse au sujet, et dans laquelle :
a) le sujet est un sujet humain et la vitamine A est destinée à être administrée au sujet à une dose de > 10 000 à 100 000 UI de vitamine A par jour ; ou
b) le sujet est un cheval et la vitamine A doit être administrée au cheval à une dose de 250 à 500 Ul/kg de poids corporel par jour.

2. Vitamine A pour utilisation selon la revendication 1, dans laquelle le sujet est un sujet humain, et la vitamine A est destinée à être administrée au sujet à une dose de 25 000 à 100 000, 50 000 à 100 000 ou 75 000 à 100 000 UI de vitamine A par jour.

3. Vitamine A pour utilisation selon la revendication 1, dans laquelle le sujet est un sujet humain adulte, et la vitamine A est destinée à être administrée au sujet à une dose de > 10 000 à 50 000 UI de vitamine A par jour, ou 25 000 à 50 000 UI de vitamine A par jour.

4. Vitamine A pour utilisation selon la revendication 1 ou la revendication 3, dans laquelle le sujet est un sujet humain, et la vitamine A doit être administrée à >10 % à 50 %, ou 25 % à 50 % d'une dose maximale sûre de vitamine A pour le sujet par jour.

5. Vitamine A pour utilisation selon la revendication 1, dans laquelle le sujet est un cheval, et la vitamine A doit être administrée à 25 % à 50 % d'une dose maximale sûre de vitamine A pour le sujet par jour.

6. Vitamine A pour utilisation selon une quelconque revendication précédente, dans laquelle la vitamine A comprend de la vitamine A isolée, une vitamine A préformée, telle qu'un ester de rétinyle ou du rétinol, une provitamine A, telle qu'un caroténoïde, ou une forme bioactive de vitamine A, telle que l'acide rétinien ou rétinoïque.

7. Vitamine A pour utilisation selon une quelconque revendication précédente pour une administration au sujet jusqu'à cinq fois par jour, pendant au moins une semaine, au moins un mois, au moins 6 mois, ou jusqu'à 6 ans à compter du jour de la première administration au sujet.

8. Vitamine A pour utilisation selon une quelconque revendication précédente, dans laquelle la vitamine A est le seul agent actif non cellulaire, non antibiotique, à administrer au sujet.

9. Formulation à doses multiples pour utilisation dans le traitement de lésions tissulaires chez un sujet humain résultant d'une blessure causée au sujet, dans laquelle la blessure comprend une blessure des tissus mous, dans laquelle la blessure des tissus mous est une blessure tendineuse ou une blessure ligamentaire, dans laquelle la formulation à doses multiples comprend une pluralité de doses unitaires distinctes de vitamine A, dans laquelle chaque dose unitaire comprend > 10 000 UI à 100 000 UI de vitamine A.

10. Formulation à doses multiples pour utilisation selon la revendication 9, dans laquelle chaque dose unitaire comprend 25 000 à 100 000, 50 000 à 100 000 ou 75 000 à 100 000 UI de vitamine A.

11. Formulation à doses multiples pour utilisation selon la revendication 9 ou la revendication 10, dans laquelle la vitamine A comprend de la vitamine A isolée, une vitamine A préformée, telle qu'un ester de rétinyle ou du rétinol, une provitamine A, telle qu'un caroténoïde, ou une forme bioactive de vitamine A, telle que l'acide rétinien ou rétinoïque.

12. Formulation à doses multiples pour utilisation selon l'une quelconque des revendications 9 à 11, dans laquelle la vitamine A fait partie d'une composition pharmaceutique comprenant de la vitamine A et un support, excipient ou diluant pharmaceutiquement acceptable.

13. Formulation à doses multiples pour utilisation selon la revendication 12, dans laquelle la vitamine A est le seul agent actif non cellulaire, non antibiotique, présent dans la composition pharmaceutique.

14. Formulation à doses multiples pour utilisation selon l'une quelconque des revendications 9 à 13 comprenant au moins 7, au moins 30, ou au moins 100 doses unitaires distinctes de vitamine A.

15. Formulation à doses multiples pour utilisation selon l'une quelconque des revendications 9 à 14, dans laquelle les doses unitaires sont destinées à une administration orale.
